# EUROPEAN PATENT SPECIFICATION

(11) **EP 4 540 828 B1**
(45) Date of publication and mention of the grant of the patent: **09.12.2026**
(21) Application number: 23732139.3
(22) Date of filing: 13.06.2023
(51) Int. Cl.: G16H 10/60, A01K 29/00, G16H 15/00

(54) **DIGITAL MONITORING OF PATHOGEN LOAD IN LIVESTOCK**
DIGITALE ÜBERWACHUNG DER PATHOGENBELASTUNG IN VIEH
SURVEILLANCE NUMÉRIQUE DE LA CHARGE PATHOGÈNE DANS LE BÉTAIL

(30) Priority: 15.06.2022 EP 22179243
(43) Date of publication of application: 23.04.2025
(73) Proprietor: Evonik Operations GmbH, 45128 Essen (DE)
(72) Inventor: IGWE, Emeka Ignatius, 80333 München (DE); LEVY, Franziska, 79379 Mülheim (DE); FECHER, Marco, 63843 Niedernberg (DE); FICKLER, Johann, 63853 Mömlingen (DE); THIEMANN, Frank, 48031 Nottuln (DE); BINGEMANN, Patrick, 67227 Frankenthal (DE)
(74) Representative: Evonik Patent Association
(86) International application number: PCT/EP2023/065782
(87) International publication number: WO 2023/242180

(56) References cited:
- JP-A- 2022 062 676
- US-A1- 2013 275 316
- US-A1- 2018 298 428

## Description

The present invention relates to a computer-implemented method for monitoring pathogen load in livestock, using a risk matrix for evaluating the risk of a pathogen load in livestock to cause a disease, a computer program product comprising code portions adapted for performing the method according to the present invention, and a system for monitoring pathogen load in livestock.

Infectious diseases of farm animals are one of the major threats to agriculture and can cause considerable damage at local, regional, and even at the international level both in industrialized and in developing countries. In the last two centuries considerable efforts have been invested in understanding the causes and pathogenesis of viral and bacterial diseases of domestic animals. These studies have introduced new methodologies for the diagnosis, treatment, and control of veterinary diseases. Importantly, research on veterinary pathogens also had a major impact in understanding basic biological processes of viruses and bacteria. In some cases, studies on veterinary pathogens have revolutionized biology and established entirely new disciplines.

The production cycle of livestock is often relatively short, in particular the production cycle of poultry. It therefore gives the farmers only a narrow time frame of opportunity for an effective intervention, especially when the risk of infectious diseases is eminent. Thus, infectious diseases can have a severe effect on the production cycle of livestock. Hence, in the first place, it needs a reliable and fast procedure for the detection of diseases-causing pathogens in livestock production, which gives the farmer a sufficient time frame of opportunity for an effective intervention.

However, the current best practice for the detection and/or monitoring of diseases-causing pathogens in livestock production involves several stand-alone processes with little or no connectivity and a time to result far exceeding the window of intervention. This practice limits the effective control of the entire process and poses some bottleneck to process standardization. Other methods like the use of sensors, cameras, and sound tracing devices assisted by artificial intelligence have also been reported to be viable approaches for monitoring livestock production processes. However, these approaches are in principle limited to the detection, estimation, and the tracking of the physical parameters, which may not be correlated with pathogen loads and/or disease occurrences of livestock farms.

Accordingly, there was still a need for a way of monitoring pathogen load in livestock, which gives the farmer a sufficient time frame of opportunity for an effective intervention.

It was found that this problem is solved by a method for monitoring pathogen load in livestock to find out the probability of a pathogen to cause a disease. In this method data being indicative for the identity and load of one or more pathogens in a livestock are evaluated by means of the risk matrix, which comprises, for each pathogen, a value for the limit of detection of the pathogen, a value for the lower limit for the occurrence of a disease caused by the pathogen, and a value for the threshold for the occurrence of a disease caused by the pathogen are comprised in a risk matrix, which allows for monitoring pathogen load in livestock. The risk matrix thus allows an assessment of the risk of the occurrence of a disease caused by a specific pathogen.

The risk matrix for evaluating the risk of a pathogen load in livestock to cause a disease,
comprises, for each pathogen, a value *v_{D}* for the limit of detection of the pathogen, a value *v_{L}* for the lower limit for the occurrence of a disease caused by the pathogen, and a value *v_{T}* for the threshold for the occurrence of a disease caused by the pathogen,
wherein
   - *v_{L}* is n × *v_{D}* with n being a real number larger than 1, wherein the value for n is given by the likelihood of the pathogen in question to cause an infectious disease,
and wherein
   - a value for the pathogen load of not more than *v_{D}* indicates that the risk for an infectious disease caused by the pathogen in question is unlikely,
   - a value for the pathogen load being larger than *v_{D}* and not more than *v_{L}* indicates that the risk for an infectious disease caused by the pathogen in question is rare,
   - a value for the pathogen load being larger than *v_{L}* and not more than *v_{T}* indicates that the risk for an infectious disease caused by the pathogen in question is possible, and
   - a value for the pathogen load being larger than *v_{T}* indicates that the risk for an infectious disease caused by the pathogen in question is certain.

One object of the present invention is therefore a computer-implemented method for monitoring pathogen load in livestock as defined by the appended claims.

Also described is a computer-implemented method for monitoring pathogen in livestock, comprising the steps of
a) receiving a request from an input/output device of a user to connect to an operating unit, wherein said request comprises the input of at least the user identity,
b) the user request of step a) initiates the operating unit to perform at least the following steps
   c) receiving or retrieving data from the input/output device of step a), wherein said data comprises at least
      C1) data *Dₚₐₜ* being indicative for the identity and load of one or more pathogens in a livestock, wherein the identity and the load of said pathogen are comprised in the data *Dₚₐₜ,*
      C2) identification *I_{farm}* of the farm, and/or farmhouse of the livestock of C1), and
      C3) the time point *T_{data}* of generation of the data of C1),
   c') determining the identity and/or load of the one or more pathogens in the data *Dₚₐₜ,* in case the identity and/or load of the pathogen is not already comprised in the data *Dₚₐₜ,*
   d) evaluating the data of C1) and/or, if applicable, of c'), comprising the steps of
      d1) pulling a risk matrix from a database,
         wherein the risk matrix comprises, for each pathogen, a value *v_{D}* for the limit of detection of the pathogen, a value *v_{L}* for the lower limit of the occurrence of a disease caused by the pathogen, and a value *v_{T}* for the threshold of the occurrence of a disease caused by the pathogen, wherein
         - *v_{L}* is *n* × *v_{D}* with *n* being a real number larger than 1, wherein the value for *n* is given by the likelihood of the pathogen in question to cause an infectious disease.
         and wherein
         - a value for the pathogen load of not more than *v_{D}* indicates that the risk for an infectious disease caused by the pathogen in question is unlikely,
         - a value for the pathogen load being larger than *v_{D}* and not more than *v_{L}* indicates that the risk for an infectious disease caused by the pathogen in question is rare,
         - a value for the pathogen load being larger than *v_{L}* and not more than *v_{T}* indicates that the risk for an infectious disease caused by the pathogen in question is possible, and
         - a value for the pathogen load being larger than *v_{T}* indicates that the risk for an infectious disease caused by the pathogen in question is certain
      d2) for every pathogen in the data *Dₚₐₜ* and/or whose identity is determined in step c'), reading off the values *v_{D}, v_{L}* and *v_{T}* from the risk matrix of step d1), and
      d3) for every pathogen, comparing the data *Dₚₐₜ* indicative for the pathogen load or the pathogen load determined in step c') with the values *v_{D}, v_{L}* and *v_{T}* of step d2) to find out the probability of any of the pathogens of C1) or identified in c') to cause a disease.

The value *v_{D}* for the limit of detection depends on the selectivity of the kit and/or the method used for the determination of the identity and the load of the pathogen in question. In addition, the individual conditions in farms or farmhouses may have an effect of varying strength on the sensitivity of the kit and/or the method used for the determination of the identity and the load of the pathogen in question. This also has an effect on the limit of detection and its value *v_{D}*. Therefore, the user of the risk matrix and of the method according to the present invention may choose *n* adequately to allow adequate and reliable conclusions for the values *v_{L}* and *v_{T}*. Accordingly, *n* is not subject to any limitations regarding its specific value. In any case *n* is a real number larger than 1, preferably *n* is a real number from larger than 1 to 10. It is preferred that the value of *n* is given by the likelihood of the pathogen in question to cause an infectious disease. Some pathogens have a larger likelihood to cause an infectious disease than other pathogens. Accordingly, the value of *n* is lower for those pathogens having a larger likelihood to cause an infectious disease, while the value of *n* is higher for those pathogens having a larger likelihood to cause an infectious disease.

The value *v_{L}* for the lower limit for the occurrence of a disease caused by the pathogen indicates the lower limit of the pathogen load for which it is typically not observed that a pathogen caused disease occurs.

The value *v_{T}* for the threshold for the occurrence of a disease caused by the pathogen indicates the threshold of the pathogen load for which it is typically observed that a pathogen caused disease occurs.

The values *v_{L}* and *v_{T}* depend on the individual pathogen in question. Some pathogens may cause diseases in livestock already at relatively low pathogen loads, while other pathogens may cause disease in livestock only at relatively high pathogen loads.

The request of step a) may further comprise the input of the identification *I_{farm}* of the farm, and/or farmhouse of the livestock.

In the context of the present invention the term livestock is used as known to the person skilled in the art and denotes the domesticated animals raised in an agricultural setting to provide labor and produce commodities such as meat, eggs, milk, fur, leather, and wool. Hence, in the context of the present invention the term livestock, both with respect to the risk matrix and the method according to the present invention, is not subject to any limitation regarding a specific type of animal. Rather, said term comprises all domesticated animals raised in an agricultural setting to provide labor and produce commodities such as meat, eggs, milk, fur, leather, and wool, e.g., cattle, sheep, goats, pigs, equine, and poultry. Preferred poultry is chicken and turkeys.

In an embodiment of the present invention the livestock is one or more of cattle, sheep, goats, pigs, equine, and poultry.

The method according to the present invention is neither limited to any specific domesticated animals or species nor to any specific pathogens or pathogenic load. Rather, the risk matrix used in the method according to the present invention may comprise a value *v_{D}* for the limit of detection of the pathogen, a value *v_{L}* for the lower limit of the occurrence of a disease caused by the pathogen, and a value *v_{T}* for the threshold of the occurrence of a disease caused by the pathogen for each pathogen causing a disease in livestock, e.g., cattle, sheep, goats, pigs, equine, and poultry. Alternatively, the risk matrix may comprise a value *v_{D}* for the limit of detection of the pathogen, a value *v_{L}* for the lower limit of the occurrence of a disease caused by the pathogen, and a value *v_{T}* for the threshold of the occurrence of a disease caused by the pathogen for each pathogen causing a disease in a specific livestock, i.e., in one of cattle, sheep, goats, pigs, equine, and poultry. In step d1) either a risk matrix for all domesticated animals or a risk matrix for the specific livestock in question is drawn from a database.

In the context of the present invention the term pathogen in the data *Dₚₐₜ* is used to denote that the identity of the pathogen and the load of said pathogen are comprised in the data *Dₚₐₜ*.

Alternatively, in case the identity of the pathogen is not comprised in the data *Dₚₐₜ,* the method further comprises the step c') determining the identity and/or load of the one or more pathogens in the data *Dₚₐₜ.*

In the context of the present invention the term pathogen is used as known to the person skilled in the art and denotes any organism that can cause a disease. The term pathogen may also be referred to as an infectious agent, or simply a germ. Roughly speaking, pathogenic organisms are of five main types: viruses, bacteria, fungi, protozoa, and worms. The pathogen may cause a disease in the livestock in the first place and/or in humans who consume any animal-derived food products, contaminated with disease causing pathogens.

The five main types of pathogens and examples for them are further disclosed:
Bacteria are ubiquitous in nature; many species perform functions essential or beneficial to animal or human life, while others cause disease. Bacteria are unicellular organisms that lack an organized nucleus and contain no chlorophyll. They can have various shapes: spherical (coccus), rod-shaped (bacillus), comma-shaped (vibrio), spiral (spirillum), or corkscrew-shaped (spirochete), and may range from 0.5 to 5.0 µm in size. Some bacteria live in soil, plants, or water; others are parasites of humans, animals, and plants. Generally, bacteria can be classified into three groups based on their need for oxygen. Aerobic bacteria thrive in the presence of oxygen and require oxygen for continued growth and existence. Anaerobic bacteria thrive in oxygen-free environments. Facultative anaerobes can survive in either environment, although they prefer the presence of oxygen.

Important pathogenic bacteria include:
*Campylobacter* (meaning "curved bacteria") is a genus of Gram-negative bacteria. Some *Campylobacter* species can infect humans, sometimes causing campylobacteriosis, a diarrheal disease in humans. The most known source for *Campylobacter* is poultry, but due to their diverse natural reservoir, *Campylobacter* spp. can also be transmitted *via* water. Other known sources of *Campylobacter* infections include food products, such as unpasteurized milk and contaminated fresh products. Sometimes the source of infection can be direct contact with infected animals, which often carry *Campylobacter* asymptomatically. At least a dozen species of *Campylobacter* have been implicated in human disease, with *C. jejuni* (80-90%) and *C. coli* (5-10%) being the most common. *C. jejuni* is recognized as one of the main causes of bacterial foodborne disease in many developed countries. It is the number one cause of bacterial gastroenteritis in Europe, with over 246,000 cases confirmed annually. *C*. *fetus* can cause spontaneous abortions in cattle and sheep and is an opportunistic pathogen in humans.

*Salmonella* is a genus of rod-shaped (bacillus) Gram-negative bacteria of the family Enterobacteriaceae. It causes salmonellosis, which is typically a food-borne disease. In humans, the most common symptoms are diarrhea, fever, abdominal cramps, and vomiting. Symptoms typically occur between 12 hours and 36 hours after exposure, and last from two to seven days. Occasionally more significant disease can result in dehydration. The old, young, and others with a weakened immune system are more likely to develop severe disease. Specific types of *Salmonella* can result in typhoid fever or paratyphoid fever. Salmonellosis is one of the most common causes of diarrhea globally. In 2015, 90,300 deaths occurred from nontyphoidal salmonellosis, and 178,000 deaths from typhoidal salmonellosis. In the United States, about 1.35 million cases and 450 deaths occur from non-typhoidal salmonellosis a year. In Europe, it is the second most common foodborne disease after campylobacteriosis.

*Clostridium perfringens* (formerly known as *C*. *welchii,* or *Bacillus welchii*) is a Gram-positive, rod-shaped, anaerobic, spore-forming pathogenic bacterium of the genus *Clostridium. C. perfringens* is ever-present in nature and can be found as a normal component of decaying vegetation, marine sediment, the intestinal tract of humans and other vertebrates, insects, and soil. *Clostridium perfringens* is one of the most common causes of food poisoning in the United States, alongside norovirus, *Salmonella, Campylobacter,* and *Staphylococcus aureus.* Infections due to *C*. *perfringens* show evidence of tissue necrosis, bacteremia, emphysematous cholecystitis, and gas gangrene, also known as clostridial myonecrosis.

*Escherichia coli* is a widely used gram-negative rod bacterium. Several non-pathogenic strains are part of the normal intestinal flora of humans. However, there are some serologically distinguishable strains that cause intestinal diseases in humans. In addition to the Enterohemorrhagic Escherichia coli (EHEC), which were first described in 1977, there are other pathogenic E. coli: enteropathogenic E. coli (EPEC), enterotoxin-forming E. coli (ETEC) and enteroinvasive E. coli (EIEC), enteroaggregative E. coli (EAEC) and diffusely adherent E. coli (DAEC).

*Enterohemorrhagic Escherichia coli* (EHEC) are certain disease-causing strains of the intestinal bacterium Escherichia coli (E. coli). The name prefix enterohemorrhagic (entero from ancient Greek 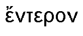 enteron - "intestine" and hemorrhagic for bleeding) indicates that EHEC can cause bloody diarrheal diseases (enterohemorrhagic colitis) in humans. It is verotoxin-producing Escherichia coli (VTEC) and synonymous with Shiga toxin-producing Escherichia coli (STEC) which cause clinical pictures in the patient. The pathogen, and the infectious diseases it causes, occur worldwide. The main reservoir of the pathogen is formed by ruminants, especially cattle, but also sheep, goats, and deer, in whose intestines they occur regularly without causing them diseases. In various countries of the world, there have been major outbreaks of EHEC, mainly due to the serotype O157:H7: For example, in 1982, when many people in the USA fell ill after eating insufficiently heated hamburgers, in 1996 in Japan with about 9000 sick schoolchildren after eating radish sprouts, and in 2006 from California in 26 states of the USA. In Germany, a continuous increase in the number of reports was observed in 2003 since the introduction of the nationwide reporting obligation in 1998.

*Enteropathogenic E. coli* (EPEC) cause severe diarrhea in young children, which is rare in industrialized societies but often responsible for childhood deaths in underdeveloped countries. Using EPEC adhesion factor (EAF), EPEC attach to the epithelial cells of the small intestine and then inject toxins into the enterocytes using a type III secretion system. There are also so-called atypical EPEC. They show the serotypes commonly used in STEC as well as virulence and pathogenicity factors such as the eae gene. However, they probably lost the Stx prophage and the associated stx genes characteristic of STEC.

*Enterotoxic E. coli* (ETEC) are more common causative agents of traveler's diarrhea ("Montezuma's revenge"). The reason for this disease is a heat-labile enterotoxin of the A/B type (LT I and LT II), as well as a heat-stable enterotoxin (ST). This 73 kDa protein has two domains, one of which binds to a G-ganglioside of the target cell (binding domain). The other domain is the active component, which activates adenylate cyclase similar to cholera toxin (about 80% gene homology). The ST, which is about 15-20 amino acids long, activates guanylate cyclase. Activation of adenylate cyclase and guanylate cyclase ends in secretory diarrhea, in which a lot of water and electrolytes are lost. The bacterium receives the genetic information from a lysogenic phage by transduction.

*Enteroinvasive E. coli* (EIEC) penetrate the epithelial cells of the colon and multiply there. Within the cell, actin tail formation occurs, with which they penetrate into neighboring epithelial cells like Listeria and Shigella. Inflammation and ulceration occur with the secretion of blood, mucus, and white blood cells (granulocytes). In addition, EIEC can release enterotoxins, which lead to electrolyte and water loss. The clinical picture is similar to bacterial dysentery with fever and bloody-mucous diarrhea, whereby a weakened symptomatology is often accompanied by watery diarrhea.

*Enteroaggregative E. coli* (EAggEC or EAEC) have the ability of auto-aggregating. They attach themselves to the small intestine epithelium with specific fimbriae. Characteristic is the increased mucus production of mucosa cells, which delays excretion. There is a diarrhea of the secretory type due to enterotoxins (EAST). EAEC causes both acute and chronic recurrent diarrheal diseases that can last for weeks. In addition to watery mucous diarrhea, there may also be fever and vomiting or bloody stools. In immunosuppressed people, e.g., HIV patients, EAEC is the most common causative agent of bacterial enteritis.

Other bacteria, however, of generally secondary role only, include Yersinia, Shigella, Brucella, and Leptospirosis.

Protozoa (singular protozoon or protozoan, plural protozoa or protozoans) is an informal term for a group of single-celled eukaryotes, either free-living or parasitic, that feed on organic matter such as other microorganisms or organic tissues and debris. Historically, protozoans were regarded as "one-celled animals", because they often possess animal-like behaviors, such as motility and predation, and lack a cell wall, as found in plants and many algae. A protozoan infection, also called protozoonosis, or protozoan disease, is the active or passive penetration of protozoa (animal single-celled organisms) into an organism, their reproduction there and the subsequent reaction of the organism in the form of a disease.

Important pathogenic protozoa include:
*Entamoeba histolytica (Amoebozoa),* whose source of transmission are water and food, causes amoebiasis.

*Balantidium coli* (Ciliate), whose source of transmission are water and food, causes balantidiasis.

*Toxoplasma gondii* (Apicomplexa), whose source of transmission is amongst others undercooked meat, causes toxoplasmosis.

*Cryptosporidium* spp. (Apicomplexa), whose source of transmission are the fecal contamination of food and water, causes *cryptosporidiosis.*

*Cyclospora cayetanensis* (Apicomplexa), whose source of transmission are the fecal contamination of food and water, causes *cyclosporiasis.*

Viruses are small infectious agents that viruses that infect all life forms, from animals and plants to microorganisms, including bacteria and archaea. Viruses are found in almost every ecosystem on earth and are the most numerous type of biological entity. When infected, a host cell is often forced to rapidly produce thousands of copies of the original virus. When not inside an infected cell or in the process of infecting a cell, viruses exist in the form of independent particles, or virions, consisting of (i) the genetic material, i.e., long molecules of DNA or RNA that encode the structure of the proteins by which the virus acts; (ii) a protein coat, the *capsid,* which surrounds and protects the genetic material; and in some cases (iii) an outside envelope of lipids. The shapes of these virus particles range from simple helical and icosahedral forms to more complex structures. Most virus species have virions too small to be seen with an optical microscope and are one-hundredth the size of most bacteria.

There is a large number of important pathogenic viruses for all domesticated animals or for some of them. Some of these viruses, diseases they cause, and the domesticated species affected by them, are listed in the table below:

**Table 1: List of exemplary viruses, the diseases they cause, and the domesticated species being affected (P. Murcia et al., "Viral Pathogens of Domestic Animals and Their Impact on Biology, Medicine and Agriculture", Encyclopedia of Microbiology 2009, 805-819).**

| **Domesticated species affected** | **Disease name** | **Virus** |
|---|---|---|
| | Aujeszky's disease | Pseudorabies virus |
| | Bluetongue | Bluetongue virus |
| | Foot-and-mouth disease | Foot-and-mouth disease virus (serotypes A, O, C, SAT1, SAT2, SAT3, Asia1) |
| | Japanese encephalitis | Japanese encephalitis virus |
| Multiple | Rabies | Rabies virus |
| | Rift Valley fever | Rift Valley fever virus |
| | Rinderpest | Rinderpest virus |
| | Vesicular stomatitis | Vesicular stomatitis virus |
| | West Nile fever | West Nile fever virus |
| Cattle | Bovine viral diarrhea | Bovine viral diarrhea virus |
| | Enzootic bovine leukosis | Bovine leukemia virus |
| | Infectious bovine rhinotracheitis/infectious pustular vulvovaginitis | *Bovine herpesvirus 1* |
| | Lumpky skin disease | Lumpky skin disease virus |
| Sheep and goats | Caprine arthritis and encephalitis | Caprine arthritis and encephalitis virus |
| | Peste des petits ruminants | *Peste-des-petits-ruminants virus* |
| | Sheeppox and goatpox | Sheeppox and goatpox viruses |
| Equine | African horse sickness | African horse sickness virus |
| | Equine encephalomyelitis (eastern) | Eastern equine encephalomyelitis virus |
| | Equine encephalomyelitis (western) | Western equine encephalomyelitis virus |
| | Equine infectious anemia | Equine infectious anemia virus |
| | Equine influenza | Equine influenza virus |
| | Equine rhinopneumonitis | *Equine herpesvirus 4* |
| | Equine viral arteritis | *Equine arteritis virus* |
| | Venezuelan equine encephalomyelitis | Venezuelan equine encephalomyelitis virus |
| Swine | African swine fever | African swine fever virus |
| | Classical swine fever | Classical swine fever virus |
| | Nipah virus encephalitis | Nipah virus |
| | Porcine reproductive and respiratory syndrome | Porcine reproductive and respiratory syndrome virus |
| | Swine vesicular disease | Swine vesicular disease virus |
| | Transmissible gastroenteritis | Transmissible gastroenteritis virus of swine |
| Avian (poultry) | Avian infectious bronchitis | Avian infectious bronchitis virus |
| | Avian infectious laryngotracheitis | Infectious laryngotracheitis virus |
| | Duck hepatitis | Duck hepatitis virus |
| | Avian influenza | High and low pathogenic avian influenza viruses |
| | Infectious bursal disease (Gumboro disease) | Infectious bursal disease virus |
| | Marek's disease | Marek's disease virus |
| | Newcastle disease | Newcastle disease virus |
| | Turkey rhinotracheitis | Avian metapneumovirus |

The method as claimed is in particular useful for poultry, because of their relatively short life cycle. When poultry is the livestock in the method according to the present invention, the pathogen is preferably one or more of *Campylobacter, Salmonella, Clostridium perfringens, Escherichia coli, Enterohemorrhagic Escherichia coli, Enteropathogenic E. coli, Enterotoxic E. coli, Enteroinvasive E. coli, Enteroaggregative E. coli,* avian infectious bronchitis virus, infectious laryngotracheitis virus, duck hepatitis virus, high and low pathogenic avian influenza viruses, infectious bursal disease virus, Marek's disease virus, Newcastle disease virus, and avian metapneumovirus.

In step c) of the described method data *Dₚₐₜ* are received or retrieved from the input/output device of step a), which are indicative for the identity and load of one or more pathogens in a livestock. Such data are preferably the concentration of a pathogen per mass, and/or the number of DNA copies of a pathogen per mass.

In an embodiment of the described method the data of C1) comprises the concentration of a pathogen per mass, and/or the number of DNA copies of a pathogen per mass. It is preferred that the probability of any of the pathogens of C1) or identified in c') to cause a disease found in step d3) is sent to the input/output device of step a).

Alternatively or additionally, it is also possible to continue working with said findings. It is beneficial to store the data *Dₚₐₜ,* the identification *I_{farm}* of the farm, and/or farmhouse, and the time point *T_{data}* of generation of the data *Dₚₐₜ,* received or retrieved in step c) together with the finding of step d) in a dataset *Dᵤₛₑᵣ,* for the user of step a). When the method of claim 1 is repeatedly carried out for the same user identity, it is beneficial to store the data *Dₚₐₜ,* the identification *I_{farm}* of the farm, and/or farm-house, and the latest time point *T_{data}* of generation of the data, received or retrieved in step c), together with the finding of step d3) in the same dataset *Dᵤₛₑᵣ* as in step c1). This allows to provide for an aggregation of relevant data, e.g., for the representation of the development of the collected *Dₚₐₜ* and the identification of the trends in the development of the pathogen load in the livestock in question.

In a further embodiment the described method further comprises the steps
d4) storing the data *Dₚₐₜ,* the identification *I_{farm}* of the farm, and/or farmhouse, and the time point *T_{data}* of generation of the data *Dₚₐₜ,* received or retrieved in step c) together with the finding of step d3) in a dataset *Dᵤₛₑᵣ,* for the user of step a), and
d5) when the method according to the present invention is repeatedly carried out for the same user identity, storing the data *Dₚₐₜ,* the identification *I_{farm}* of the farm, and/or farmhouse, and the latest time point *T_{data}* of generation of the data, received or retrieved in step c), together with the finding of step d3) in the same dataset *Dᵤₛₑᵣ* as in step c1).

It is further beneficial that for each farm and/or farmhouse of the user of step a) the data *Dₚₐₜ,* findings and latest time point *T_{data}* are grouped as individual reports in a list, with the reports having the latest time points at the top of the list. This gives the user a complete overview over the situation in each of his farms and/or farmhouses at the time points of data generation.

In another embodiment the described method further comprises the steps
e) the user request of step a) initiates the operating unit to perform at least the steps
e1) for each farm and/or farmhouse of the user of step a), pulling the data *Dₚₐₜ,* the findings and the latest time point *T_{data}* of generation of the data stored in step d4) or d5) from the dataset *Dᵤₛₑᵣ* for said user,
e2) for each farm and/or farmhouse, grouping the data *Dₚₐₜ,* findings and latest time point *T_{data}* pulled in step e1) as individual reports in a list, with the reports having the latest time points *T_{data}* at the top of the list, and
e3) sending the list of reports obtained in step e2) to the input/output device of the user.

Preferably, the reports indicate the probability of a pathogen in question to cause a risk according to the risk matrix according to the present invention. It is further preferred that, for every pathogen in question, i.e., in the data *Dₚₐₜ,* the risk for an infectious disease caused by the pathogen in question is categorized as
- unlikely, when the data *Dₚₐₜ* being indicative for the load of the pathogen is not more than *v_{D}* for said pathogen in the risk matrix,
- rare, when the data *Dₚₐₜ* being indicative for the load of the pathogen is larger than *v_{D}* and not more than *v_{L}* for said pathogen in the risk matrix,
- possible, when the data *Dₚₐₜ* being indicative for the load of the pathogen is larger than *v_{L}* and not more than *v_{T}* for said pathogen in the risk matrix, and
- certain, when the data *Dₚₐₜ* being indicative for the load of the pathogen is larger than *v_{T}* for said pathogen in the risk matrix.

In general, each value, in particular any measured value, is subject to a measurement error or an inaccuracy in the method of determination. The described method therefore has to consider any measurement error or inaccuracies in the method of determination, when assessments are made regarding the risk for an infectious disease without losing any validity in the assessments. This is in particular relevant when the pathogen load is very close to the values *v_{L}* and *v_{T}* and the risk for an infectious disease caused by the pathogen in question could be either categorized as possible or certain, which makes a big difference. It was found that this problem is solved by considering a p-value of 0.05 as statistically relevant difference from the values *v_{L}* and *v_{T}.* In detail, the risk for an infectious disease caused by the pathogen in question is categorized in the next higher risk class, when the p-value from the comparison of the load for the pathogen in question and the reference value for the lower risk class is < 0.05. For example, the load for the pathogen in question is categorized as certain, when said load has a p-value of < 0.05 compared to the value *v_{T}.*

It is preferred that the identity and the load of the one or more pathogen in question are given in specific colors indicating the degree of risk or in a specific pattern according to a code indicating the degree of risk. This has the advantage of a better awareness of any potential risk for the occurrence of a pathogen caused disease. For example,
- when the risk for an infectious disease caused by the pathogen in question is unlikely, the identity of the pathogen, and/or the data *Dₚₐₜ* being indicative for its load are colored in green,
- when the risk for an infectious disease caused by the pathogen in question is rare, the identity of the pathogen, and/or the data *Dₚₐₜ* being indicative for its load are colored in yellow,
- when the risk for an infectious disease caused by the pathogen in question is possible, the identity of the pathogen, and/or the data *Dₚₐₜ* being indicative for its load are colored in orange, and
- when the risk for an infectious disease caused by the pathogen in question is certain, the identity of the pathogen, and/or the data *Dₚₐₜ* being indicative for its load are colored in red.

In the next step, the user can select from the thus provided list of reports. For the report selected in step e4), the load of the livestock with every pathogen in the data *Dₚₐₜ* for each time point *T_{data}* of generation of the data *Dₚₐₜ* is plotted into a diagram and for every pathogen in data *Dₚₐₜ,* the values *v_{L}* and *v_{T}* of the occurrence of the disease caused by the pathogen is also plotted into the diagram. Since the data are plotted into the diagram for each time point of data generation, this procedure facilitates the identification of trends in the development of the pathogen load in livestock both in general, i.e., the overall pathogen load, and in particular, i.e., the specific load with a specific pathogen. Further, the overall risk of the occurrence can be identified when the value(s) *v_{L}* and/or *v_{T}* read off from the risk matrix also is/are plotted into the diagram.

In a preferred embodiment step e) of the described method further comprises the steps
e4) receiving a selection of a report from the input/output device of the user,
e5) for the report selected in step e4), plotting the load of the livestock with every pathogen in the data *Dₚₐₜ* for each time point *T_{data}* of generation of the data *Dₚₐₜ* into a diagram,
e6) pulling the risk matrix according to claim 1, preferably the risk matrix of step
   d1) of the method according to the claims, from a database,
e7) for every pathogen in the data *Dₚₐₜ,* reading off the value(s) *v_{L}* and/or *v_{T}* for the one or more pathogens from the risk matrix according to claim 1, preferably the risk matrix of step d1) of the method according to the claims, and
e8) plotting the value(s) *v_{L}* and/or *v_{T}* read off in step e7) into the diagram obtained in step e5).

Once the user has read the selected report, the user is typically no longer interested in reading the same old reports again. Rather, the user's main focus is on the most recent reports and data. In order to avoid that the user has to go through any old reports again, the described method receives a read confirmation for the report selected in step e4) from the input/output device of the user and stores the read confirmation for the report together with the corresponding data. When at least the step e), or the described method, is carried out repeatedly, the read confirmation is also pulled from the dataset *Dᵤₛₑᵣ,* and all reports without a read confirmation are ranked in a first list, with the reports having the latest time points at the top of said first list, and all other reports are ranked in a second list, with the reports having the latest time points at the top of said second list.

In a further preferred embodiment, the step e) of the described method further comprises the steps
e9) if applicable, receiving a read confirmation for the report selected in step e4) from the input/output device of the user, and storing the read confirmation for the report together with the corresponding *Dₚₐₜ,* findings and latest time point *T_{data}* in the dataset *Dᵤₛₑᵣ,* and
e10) when at least the step e) is carried out repeatedly, the step e1) further comprises pulling a read confirmation from the dataset *Dᵤₛₑᵣ,* and the step e2) further comprises ranking all reports without a read confirmation in a first list, with the reports having the latest time points at the top of said first list, and all other reports in a second list, with the reports having the latest time points at the top of said second list.

It also beneficial for the user that the described method give advice for the further samplings, specifically giving advice for the date for scheduled samplings, based on the dates of time point *T_{data}* of C3).

In yet another preferred embodiment of the described method the report of step e2) further comprises the dates for scheduled samplings based on the dates of time point *T_{data}* of C3).

In still another preferred embodiment the described method further comprises the step
f) planning a new cycle for monitoring the pathogen load in livestock, comprising the steps of
f1) receiving a selection or the input of the farm and/or farmhouse for which a new cycle is to be planned,
f2) receiving a selection or the input of the start date for the new cycle, and
f3) receiving a section or the input of the livestock age at the time of starting the new cycle and the scope of the new cycle.

In order to avoid any overlaps of new cycles for monitoring the pathogen load in livestock with the cycles for monitoring the pathogen load in livestock which are active, it is beneficial to indicate these active cycles in step f2).

In a preferred embodiment the step f2) further comprises the indication of the cycles for monitoring the pathogen load in livestock which are active.

It may be also beneficial for the user to get information on the pathogen load for all farms and/or farmhouses of him, for example the mean pathogen status for all farms and/or farmhouses and the mean condition risk for all of his harms and/or farmhouses.

Preferably, the described method therefore further comprises the step
g) receiving a request from the input/output device of a user to aggregate reports, comprising the steps of
g1) for all farms and/or farmhouses of the user, pulling the load of the livestock with each pathogen in each farm and/or farmhouse from the data *Dₚₐₜ* from the dataset *Dᵤₛₑᵣ,* forming the mean of the load with each pathogen, to get the mean pathogen status for all farms and/or farmhouses.

To get the mean condition risk for all farms and/or farmhouses, the step g1) is preferably followed by the following steps
g2) pulling the risk matrix according to the present invention, in particle the risk matrix of step d1) of the method according to the present invention, from a database, and
g3) for all farms and/or farmhouses, reading off the values *v_{L}* and/or *v_{T}* for each pathogen in data *Dₚₐₜ,* and forming the mean of the values *v_{L}* and/or *v_{T}* to get the mean condition risk *v_{L,mean}* and/or *v_{T,mean}* for all farms and/or farmhouses.

The steps g1) to g3) can also be combined in one embodiment.

In another embodiment the described method further comprises the step
g) receiving a request from the input/output device of a user to aggregate reports, comprising the steps of
g1) for all farms and/or farmhouses of the user, pulling the load of the livestock with each pathogen in each farm and/or farmhouse from the data *Dₚₐₜ* from the dataset *Dᵤₛₑᵣ,* forming the mean of the load with each pathogen, to get the mean pathogen status for all farms and/or farmhouses,
g2) pulling the risk matrix according to claim 1, preferably the risk matrix of step d1) of the method according to the present invention, from a database, and
g3) for all farms and/or farmhouses, reading off the values *v_{L}* and/or *v_{T}* for each pathogen in data *Dₚₐₜ,* and forming the mean of the values *v_{L}* and/or *v_{T}* to get the mean condition risk *v_{L,mean}* and/or *v_{T,mean}* for all farms and/or farmhouses.

The method according to the claims can be used on any conceivable computer device that allows to perform said method. The objects of the present invention are not subject to any limitation regarding the computer device, provided that it allows for receiving the request of step a), receiving or retrieving the data of step c), and comprising or having access to the database with the risk matrix of the method according to the present invention. For example, the computer device can be a stationary or mobile computer. Examples for a stationary computer are a desktop computer and a network computer. Examples for a portable computer are a laptop, a notebook, a tablet, and a smartphone.

A further object of the present invention is therefore a computer program product comprising code portions adapted for performing the method according to the claims, when said program is loaded into a computer device.

Preferably, the computer program product comprising code portions either comprises or has access to a database with the risk matrix as defined in claim 1.

Alternatively, the program for the method according to the claims and/or code portions for performing said method can be stored on a computer usable medium, such as a USB stick, a memory card or a CD-ROM. In that case, the program and/or the code portions is/are stored as computer readable program means which causes a computer to perform the method as claimed.

Yet another object of the present invention is therefore a computer program product stored on a computer usable medium, comprising computer readable program means for causing a computer to perform the method as claimed.

Preferably, the computer program product stored on a computer usable medium either comprises or has access to a database with the risk matrix as defined in claim 1.

The method according to the claims can be implemented to give a system for monitoring pathogen load in livestock.

An object of the present invention is therefore also a system for monitoring pathogen load in livestock, comprising an operating unit adapted to perform the method according to the claims, and comprising or having access to the database with the risk matrix of the method according to the claims, preferably the matrix of step d1) of the method according to the claims.

## Claims

1. A computer-implemented method for monitoring pathogen load in livestock, comprising the steps of
a) receiving a request from an input/output device of a user to connect to an operating unit, wherein said request comprises the input of at least the user identity,
b) the user request of step a) initiates the operating unit to perform at least the following steps
c) receiving or retrieving data from the input/output device of step a), wherein said data comprises at least
C1) data *Dₚₐₜ* being indicative for the identity and load of one or more pathogens in a livestock, wherein the identity and the load of said pathogen are comprised in the data *Dₚₐₜ,*
C2) identification *I_{farm}* of the farm, and/or farmhouse of the livestock of C1), and
C3) the time point *T_{data}* of generation of the data of C1),
d) evaluating the data of C1), comprising the steps of
d1) pulling a risk matrix from a database,
wherein the risk matrix comprises, for each pathogen, a value *v_{D}* for the limit of detection of the pathogen, a value *v_{L}* for the lower limit of the occurrence of a disease caused by the pathogen, and a value *v_{T}* for the threshold of the occurrence of a disease caused by the pathogen, wherein
- *v_{L}* is n × *v_{D}* with *n* being a real number larger than 1, wherein the value for *n* is given by the likelihood of the pathogen in question to cause an infectious disease
and wherein
- a value for the pathogen load of not more than *v_{D}* indicates that the risk for an infectious disease caused by the pathogen in question is unlikely,
- a value for the pathogen load being larger than *v_{D}* and not more than *v_{L}* indicates that the risk for an infectious disease caused by the pathogen in question is rare,
- a value for the pathogen load being larger than *v_{L}* and not more than *v_{T}* indicates that the risk for an infectious disease caused by the pathogen in question is possible, and
- a value for the pathogen load being larger than *v_{T}* indicates that the risk for an infectious disease caused by the pathogen in question is certain
d2) for every pathogen in the data *Dₚₐₜ,* reading off the values *v_{D}, v_{L}* and *v_{T}* from the risk matrix of step d1),
d3) for every pathogen, comparing the data *Dₚₐₜ* indicative for the pathogen load with the values *v_{D}, v_{L}* and *v_{T}* of step d2) to find out the probability of any of the pathogens of C1) to cause a disease,
d4) storing the data *Dₚₐₜ,* the identification *I_{farm}* of the farm, and/or farmhouse, and the time point *T_{data}* of generation of the data *D_{Pat},* received or retrieved in step c) together with the finding of step d3) in a dataset *Dᵤₛₑᵣ,* for the user of step a),
d5) when the method is repeatedly carried out for the same user identity, storing the data *Dₚₐₜ,* the identification *I_{farm}* of the farm, and/or farmhouse, and the latest time point *T_{data}* of generation of the data, received or retrieved in step c), together with the finding of step d3) in the same dataset *Dᵤₛₑᵣ* as in step d4),
e) the user request of step a) initiates the operating unit to perform at least the steps
e1) for each farm and/or farmhouse of the user of step a), pulling the data *Dₚₐₜ,* the findings and the latest time point *T_{data}* of generation of the data stored in step d4) or d5) from the dataset *Dᵤₛₑᵣ* for said user,
e2) for each farm and/or farmhouse, grouping the data *Dₚₐₜ,* findings and latest time point *T_{data}* pulled in step e1) as individual reports in a list, with the reports having the latest time points *T_{data}* at the top of the list, and
e3) sending the list of reports obtained in step e2) to the input/output device of the user,
e4) receiving a selection of a report from the input/output device of the user,
e5) for the report selected in step e4), plotting the load of the livestock with every pathogen in the data *Dₚₐₜ* for each time point *T_{data}* of generation of the data *Dₚₐₜ* into a diagram,
e6) pulling the risk matrix of step d1) from a database,
e7) for every pathogen in the data *Dₚₐₜ,* reading off the value(s) *v_{L}* and/or *v_{T}* for the one or more pathogens from the risk matrix of step d1), and
e8) plotting the value(s) *v_{L}* and/or *v_{T}* read off in step e7) into the diagram obtained in step e5).

2. The method according to claim 1, wherein the livestock is one or more of cattle, sheep, goats, pigs, equine, and poultry.

3. The method according to claim 1 or 2, wherein the data of C1) comprises the concentration of a pathogen per mass, and/or the number of DNA copies of a pathogen per mass.

4. The method according to any of claims 1 to 3, wherein the step e) of the method according to the present invention further comprises the steps
e9) if applicable, receiving a read confirmation for the report selected in step e4) from the input/output device of the user, and storing the read confirmation for the report together with the corresponding *Dₚₐₜ,* findings and latest time point *T_{data}* in the dataset *Dᵤₛₑᵣ,* and
e10) when at least the step e) is carried out repeatedly, the step e1) further comprises pulling a read confirmation from the dataset *Dᵤₛₑᵣ,* and the step e2) further comprises ranking all reports without a read confirmation in a first list, with the reports having the latest time points at the top of said first list, and all other reports in a second list, with the reports having the latest time points at the top of said second list.

5. The method according to any of claims 1 to 4, wherein the report of step e2) further comprises the dates for scheduled samplings based on the dates of time point *T_{data}* of C3).

6. The method according to any of claims 1 to 5, further comprising the step
f) planning a new cycle for monitoring the pathogen load in livestock, comprising the steps of
f1) receiving a selection or the input of the farm and/or farmhouse for which a new cycle is to be planned,
f2) receiving a selection or the input of the start date for the new cycle, and
f3) receiving a section or the input of the livestock age at the time of starting the new cycle and the scope of the new cycle.

7. The method according to claim 6, wherein the step f2) further comprises the indication of the cycles for monitoring the pathogen load in livestock which are active.

8. The method according to any of claims 1 to 7, further comprising the step
g) receiving a request from the input/output device of a user to aggregate reports, comprising the steps of
g1) for all farms and/or farmhouses of the user, pulling the load of the livestock with each pathogen in each farm and/or farmhouse from the data *Dₚₐₜ* from the dataset *Dᵤₛₑᵣ,* forming the mean of the load with each pathogen, to get the mean pathogen status for all farms and/or farmhouses.

9. The method according to any of claims 1 to 8, further comprising the step
g) receiving a request from the input/output device of a user to aggregate reports, comprising the steps of
g1) for all farms and/or farmhouses of the user, pulling the load of the livestock with each pathogen in each farm and/or farmhouse from the data *Dₚₐₜ* from the dataset *Dᵤₛₑᵣ,* forming the mean of the load with each pathogen, to get the mean pathogen status for all farms and/or farmhouses,
g2) pulling the risk matrix of claim 1 from a database, and
g3) for all farms and/or farmhouses, reading off the values *v_{L}* and/or *v_{T}* for each pathogen in data *Dₚₐₜ,* and forming the mean of the values *v_{L}* and/or *v_{T}* to get the mean condition risk *v_{L,mean}* and/or *v_{T,mean}* for all farms and/or farmhouses.

10. A computer program product comprising code portions adapted for performing the method according to any of claims 1 to 9, when said program is loaded into a computer device.

11. A computer program product stored on a computer usable medium, comprising computer readable program means for causing a computer to perform the method according to any of claims 1 to 9.

12. A system for monitoring pathogen load in livestock, comprising an operating unit adapted to perform the method according to any of claims 1 to 9, and comprising or having access to the database with the risk matrix according to claim 1.

## Patentansprüche

1. Computerimplementiertes Verfahren zur Überwachung der Pathogenbelastung bei Nutztieren, umfassend die folgenden Schritte:
a) Empfangen einer Anforderung von einer Eingabe/Ausgabe-Vorrichtung eines Benutzers, eine Verbindung mit einer Bedieneinheit herzustellen, wobei die Anforderung die Eingabe mindestens der Benutzeridentität umfasst,
b) wobei die Benutzeranforderung von Schritt a) die Bedieneinheit veranlasst, zumindest die folgenden Schritte auszuführen:
c) Empfangen oder Abrufen von Daten von der Eingabe/Ausgabe-Vorrichtung von Schritt a), wobei die Daten mindestens Folgendes umfassen:
C1) Daten Dₚₐₜ, die die Identität und die Belastung durch ein oder mehrere Pathogene bei Nutztieren anzeigen, wobei die Identität und die Belastung durch das Pathogen in den Daten Dₚₐₜ umfasst sind,
C2) die Identifikation I_{farm} des landwirtschaftlichen Betriebs und/oder Bauernhofs der Nutztiere von C1), und
C3) den Zeitpunkt T_{data} der Erzeugung der Daten von C1),
d) Auswerten der Daten von C1), umfassend die folgenden Schritte:
d1) Abrufen einer Risikomatrix aus einer Datenbank,
wobei die Risikomatrix für jedes Pathogen einen Wert v_{D} für die Nachweisgrenze des Pathogens, einen Wert v_{L} für die untere Grenze des Auftretens einer durch das Pathogen verursachten Krankheit und einen Wert v_{T} für die Schwelle des Auftretens einer durch das Pathogen verursachten Krankheit umfasst, wobei
- V_{L} ist n x v_{D}, wobei n eine reelle Zahl größer als 1 ist, wobei der Wert für n durch die Wahrscheinlichkeit gegeben ist, dass das betreffende Pathogen eine Infektionskrankheit verursacht,
und wobei
- ein Wert für die Pathogenbelastung von nicht mehr als v_{D} anzeigt, dass das Risiko für eine durch das betreffende Pathogen verursachte Infektionskrankheit unwahrscheinlich ist;
- ein Wert für die Pathogenbelastung, der größer als v_{D} und nicht größer als v_{L} ist, anzeigt, dass das Risiko für eine durch das betreffende Pathogen verursachte Infektionskrankheit selten ist;
- ein Wert für die Pathogenbelastung, der größer als v_{L} und nicht größer als v_{T} ist, anzeigt, dass das Risiko für eine durch das betreffende Pathogen verursachte Infektionskrankheit möglich ist, und
- ein Wert für die Pathogenbelastung, der größer als v_{T} ist, anzeigt, dass das Risiko für eine durch das betreffende Pathogen verursachte Infektionskrankheit sicher ist,
d2) für jedes Pathogen in den Daten Dₚₐₜ Ablesen der Werte v_{D}, v_{L} und v_{T} aus der Risikomatrix von Schritt d1),
d3) für jedes Pathogen Vergleichen der Daten Dₚₐₜ, die die Pathogenbelastung anzeigen, mit den Werten v_{D}, v_{L} und v_{T} von Schritt d2), um die Wahrscheinlichkeit zu ermitteln, dass eines der Pathogene von C1) eine Krankheit verursacht,
d4) Speichern der Daten Dₚₐₜ, der Identifikation I_{farm} des landwirtschaftlichen Betriebs und/oder Bauernhofs und des Zeitpunkts T_{data} der Erzeugung der Daten D_{Pat}, die in Schritt c) empfangen oder abgerufen wurden, zusammen mit dem Ergebnis von Schritt d3) in einem Datensatz dᵤₛₑᵣ für den Benutzer von Schritt a),
d5) wenn das Verfahren für dieselbe Benutzeridentität wiederholt durchgeführt wird, Speichern der Daten Dₚₐₜ, der Identifikation I_{farm} des landwirtschaftlichen Betriebs und/oder Bauernhofs und des letzten Zeitpunkts T_{data} der Erzeugung der Daten, die in Schritt c) empfangen oder abgerufen wurden, zusammen mit dem Ergebnis von Schritt d3) in demselben Datensatz dᵤₛₑᵣ wie in Schritt d4),
e) wobei die Benutzeranforderung von Schritt a) die Bedieneinheit veranlasst, zumindest die folgenden Schritte auszuführen:
e1) für jeden landwirtschaftlichen Betrieb und/oder Bauernhof des Benutzers von Schritt a) Abrufen der Daten Dₚₐₜ, der Ergebnisse und des letzten Zeitpunkts T_{data} der Erzeugung der Daten, die in Schritt d4) oder d5) gespeichert wurden, aus dem Datensatz Dᵤₛₑᵣ für den Benutzer,
e2) für jeden landwirtschaftlichen Betrieb und/oder Bauernhof Gruppieren der Daten Dₚₐₜ, der Ergebnisse und des letzten Zeitpunkts T_{data}, die in Schritt e1) abgerufen wurden, als Einzelberichte in einer Liste, wobei die Berichte mit den letzten Zeitpunkten T_{data} oben in der Liste stehen, und
e3) Senden der Liste der in Schritt e2) erhaltenen Berichte an die Eingabe/Ausgabe-Vorrichtung des Benutzers,
e4) Empfangen einer Auswahl eines Berichts von der Eingabe-/Ausgabevorrichtung des Benutzers,
e5) für den in Schritt e4) ausgewählten Bericht Darstellen der Belastung der Nutztiere mit jedem Pathogen in den Daten Dₚₐₜ für jeden Zeitpunkt T_{data} der Erzeugung der Daten Dₚₐₜ in einem Diagramm,
e6) Abrufen der Risikomatrix von Schritt d1) aus einer Datenbank,
e7) für jedes Pathogen in den Daten Dₚₐₜ Ablesen des/der Werte(s) v_{L} und/oder v_{T} für das eine oder die mehreren Pathogene aus der Risikomatrix von Schritt d1) und
e8) Darstellen des/der in Schritt e7) abgelesenen Werte(s) v_{L} und/oder v_{T} in dem in Schritt e5) erhaltenen Diagramm.

2. Verfahren nach Anspruch 1, wobei es sich bei den Nutztieren um eines oder mehrere von Rindern, Schafen, Ziegen, Schweinen, Pferden und Geflügel handelt.

3. Verfahren nach Anspruch 1 oder 2, wobei die Daten von C1) die Konzentration eines Pathogens pro Masse und/oder die Anzahl von DNA-Kopien eines Pathogens pro Masse umfassen.

4. Verfahren nach einem der Ansprüche 1 bis 3, wobei Schritt e) des erfindungsgemäßen Verfahrens ferner die folgenden Schritte umfasst:
e9) gegebenenfalls Empfangen einer Lesebestätigung für den in Schritt e4) ausgewählten Bericht von der Eingabe-/Ausgabevorrichtung des Benutzers und Speichern der Lesebestätigung für den Bericht zusammen mit den entsprechenden Dₚₐₜ, Ergebnissen und letzten Zeitpunkten T_{data} im Datensatz Dᵤₛₑᵣ, und
e10) wobei, wenn zumindest Schritt e) wiederholt ausgeführt wird, Schritt e1) ferner Abrufen einer Lesebestätigung aus dem Datensatz Dᵤₛₑᵣ umfasst und Schritt e2) ferner Reihen aller Berichte ohne Lesebestätigung in einer ersten Liste mit den Berichten mit den letzten Zeitpunkten oben in der ersten Liste und aller anderen Berichte in einer zweiten Liste mit den Berichten mit den letzten Zeitpunkten oben in der zweiten Liste umfasst.

5. Verfahren nach einem der Ansprüche 1 bis 4, wobei der Bericht von Schritt e2) ferner die Daten für geplante Probenahmen basierend auf den Daten des Zeitpunkts T_{data} von C3) umfasst.

6. Verfahren nach einem der Ansprüche 1 bis 5, ferner umfassend den folgenden Schritt:
f) Planen eines neuen Zyklus zum Überwachen der Pathogenbelastung bei Nutztieren, umfassend die folgenden Schritte:
f1) Empfangen einer Auswahl oder der Eingabe des landwirtschaftlichen Betriebs und/oder Bauernhofs, für den ein neuer Zyklus geplant werden soll,
f2) Empfangen einer Auswahl oder der Eingabe des Startdatums für den neuen Zyklus und
f3) Empfangen einer Auswahl oder der Eingabe des Nutztieralters zum Zeitpunkt des Starts des neuen Zyklus und des Umfangs des neuen Zyklus.

7. Verfahren nach Anspruch 6, wobei Schritt f2) ferner die Anzeige der Zyklen zum Überwachen der Pathogenbelastung bei Nutztieren umfasst, die aktiv sind.

8. Verfahren nach einem der Ansprüche 1 bis 7, ferner umfassend den folgenden Schritt:
g) Empfangen einer Anforderung von der Eingabe-/Ausgabevorrichtung eines Benutzers zum Aggregieren von Berichten, umfassend die folgenden Schritte:
g1) für alle landwirtschaftlichen Betriebe und/oder Bauernhöfe des Benutzers Abrufen der Belastung der Nutztiere mit jedem Pathogen in jedem landwirtschaftlichen Betrieb und/oder Bauernhof aus den Daten Dₚₐₜ aus dem Datensatz Dᵤₛₑᵣ, Bilden des Mittelwerts der Belastung mit jedem Pathogen, um den mittleren Pathogenstatus für alle landwirtschaftlichen Betriebe und/oder Bauernhöfe zu erhalten.

9. Verfahren nach einem der Ansprüche 1 bis 8, ferner umfassend den folgenden Schritt:
g) Empfangen einer Anforderung von der Eingabe-/Ausgabevorrichtung eines Benutzers zum Aggregieren von Berichten, umfassend die folgenden Schritte:
g1) für alle landwirtschaftlichen Betriebe und/oder Bauernhöfe des Benutzers Abrufen der Belastung der Nutztiere mit jedem Pathogen in jedem landwirtschaftlichen Betrieb und/oder Bauernhof aus den Daten Dₚₐₜ aus dem Datensatz Dᵤₛₑᵣ, Bilden des Mittelwerts der Belastung mit jedem Pathogen, um den mittleren Pathogenstatus für alle landwirtschaftlichen Betriebe und/oder Bauernhöfe zu erhalten,
g2) Abrufen der Risikomatrix nach Anspruch 1 aus einer Datenbank und
g3) für alle landwirtschaftlichen Betriebe und/oder Bauernhöfe Ablesen der Werte v_{L} und/oder v_{T} für jedes Pathogen in den Daten Dₚₐₜ und Bilden des Mittelwerts der Werte v_{L} und/oder v_{T}, um das mittlere Zustandsrisiko v_{L, mean} und/oder v_{T, mean} für alle landwirtschaftlichen Betriebe und/oder Bauernhöfe zu erhalten.

10. Computerprogrammprodukt, umfassend Codeabschnitte, die zum Durchführen des Verfahrens nach einem der Ansprüche 1 bis 9 ausgelegt sind, wenn das Programm in eine Computervorrichtung geladen wird.

11. Computerprogrammprodukt, das auf einem computernutzbaren Medium gespeichert ist und computerlesbare Programmanweisungen umfasst, um einen Computer zum Durchführen des Verfahrens nach einem der Ansprüche 1 bis 9 zu veranlassen.

12. System zum Überwachen der Pathogenbelastung bei Nutztieren, umfassend eine Bedieneinheit, die dazu ausgelegt ist, das Verfahren nach einem der Ansprüche 1 bis 9 durchzuführen, und die die Datenbank mit der Risikomatrix nach Anspruch 1 umfasst oder Zugriff darauf hat.

## Revendications

1. Procédé mis en œuvre par ordinateur pour surveiller la charge pathogène dans le bétail, comprenant l'étape consistant à
a) recevoir une demande en provenance d'un dispositif d'entrée/de sortie d'un utilisateur de se connecter à une unité opérationnelle, ladite demande comprenant l'entrée d'au moins l'identité d'utilisateur,
b) la demande de l'utilisateur de l'étape a) amenant l'unité opérationnelle à effectuer au moins les étapes consistant à
c) recevoir ou récupérer des données à partir du dispositif d'entrée/de sortie de l'étape a), lesdites données comprenant au moins
C1) des données Dₚₐₜ indiquant l'identité et la charge d'un ou de plusieurs agents pathogènes dans le bétail, l'identité et la charge dudit agent pathogène étant comprises dans les données Dₚₐₜ,
C2) l'identification I_{farm} de l'exploitation agricole, et/ou de la ferme du bétail de C1), et
C3) le point temporel T_{data} de génération des données de C1),
d) évaluer les données de C1) comprenant les étapes consistant à
d1) extraire une matrice de risque d'une base de données,
la matrice de risque comprenant, pour chaque agent pathogène, une valeur v_{D} pour la limite de détection de l'agent pathogène, une valeur v_{L} pour la limite inférieure d'occurrence d'une maladie causée par l'agent pathogène, et une valeur v_{T} pour le seuil d'occurrence d'une maladie causée par l'agent pathogène,
- v_{L} étant n × v_{D}, n étant un nombre réel supérieur à 1, la valeur de n étant donnée par la probabilité que l'agent pathogène en question provoque une maladie infectieuse
et
- une valeur pour la charge pathogène inférieure ou égale à v_{D} indiquant que le risque de maladie infectieuse causé par l'agent pathogène en question est improbable,
- une valeur pour la charge pathogène supérieure à v_{D} et inférieure ou égale à v_{L} indiquant que le risque de maladie infectieuse causé par l'agent pathogène en question est rare,
- une valeur pour la charge pathogène supérieure à v_{L} et inférieure ou égale à v_{T} indiquant que le risque d'une maladie infectieuse causée par l'agent pathogène en question est possible, et
- une valeur pour la charge pathogène supérieure à v_{T} indiquant que le risque pour une maladie infectieuse causée par l'agent pathogène en question est certain d2) pour tout agent pathogène dans les données Dₚₐₜ, lire les valeurs v_{D}, v_{L} et v_{T} à partir de la matrice de risque de l'étape d1),
d3) pour chaque agent pathogène, comparer les données Dₚₐₜ indiquant la charge pathogène aux valeurs v_{D}, v_{L} et v_{T} de l'étape d2) afin de déterminer la probabilité qu'un quelconque des agents pathogènes de C1) provoque une maladie,
d4) stocker les données Dₚₐₜ, l'identification I_{farm} de l'exploitation agricole, et/ou de la ferme, et le point temporel T_{data} de génération des données D_{Pat} reçues ou récupérées à l'étape c) ainsi que l'établissement de l'étape d3) dans un ensemble de données Dᵤₛₑᵣ, pour l'utilisateur de l'étape a),
d5) lorsque le procédé est mis en œuvre de façon répétée pour la même identité d'utilisateur, stocker les données Dₚₐₜ, l'identification I_{farm} de l'exploitation agricole, et/ou de la ferme, et le dernier point temporel T_{data} de génération des données reçues ou récupérées à l'étape c), ainsi que l'établissement de l'étape d3) dans le même ensemble de données Dᵤₛₑᵣ qu'à l'étape d4),
e) la demande de l'utilisateur de l'étape a) amenant l'unité opérationnelle à effectuer au moins les étapes consistant à
e1) pour chaque exploitation agricole et/ou ferme de l'utilisateur de l'étape a), extraire les données Dₚₐₜ, les résultats et le dernier point temporel T_{data} de génération des données mémorisées à l'étape d4) ou d5) à partir de l'ensemble de données Dᵤₛₑᵣ pour ledit utilisateur,
e2) pour chaque exploitation agricole et/ou ferme, regrouper les données Dₚₐₜ, les résultats et le dernier point temporel T_{data} extrait à l'étape e1) sous forme de rapports individuels dans une liste, les rapports ayant les derniers points temporels T_{data} en haut de la liste, et
e3) envoyer au dispositif d'entrée/de sortie de l'utilisateur la liste de rapports obtenus à l'étape e2), e4) recevoir une sélection d'un rapport en provenance du dispositif d'entrée/de sortie de l'utilisateur,
e5) pour le rapport sélectionné à l'étape e4), tracer, dans un diagramme, la charge du bétail avec tout agent pathogène, dans les données Dₚₐₜ pour chaque temps T_{data} de génération des données Dₚₐₜ,
e6) extraire d'une base de données la matrice de risque de l'étape d1),
e7) pour chaque agent pathogène dans les données Dₚₐₜ, lire la ou les valeurs v_{L} et/ou v_{T} pour le ou les agents pathogènes de la matrice de risque de l'étape d1), et
e8) tracer la ou les valeurs v_{L} et/ou v_{T} lues à l'étape e7) dans le diagramme obtenu à l'étape e5).

2. Procédé selon la revendication 1, le bétail étant un ou plusieurs parmi les bovins, les moutons, les chèvres, les porcs, les équidés et la volaille.

3. Procédé selon la revendication 1 ou 2, les données de C1) comprenant la concentration d'un agent pathogène par masse, et/ou le nombre de copies d'ADN d'un agent pathogène par masse.

4. Procédé selon l'une quelconque des revendications 1 à 3, l'étape e) du procédé selon la présente invention comprenant en outre les étapes consistant à
e9) le cas échéant, recevoir du dispositif d'entrée/de sortie de l'utilisateur une confirmation de lecture pour le rapport sélectionné à l'étape e4), et stocker la confirmation de lecture pour le rapport avec les Dₚₐₜ, les résultats et dernier point temporel T_{data} correspondants dans l'ensemble de données Dᵤₛₑᵣ, et
e10) lorsqu'au moins l'étape e) est exécutée de façon répétée, l'étape e1) comprenant en outre l'étape consistant à extraire une confirmation de lecture de l'ensemble de données Dᵤₛₑᵣ, et l'étape e2) comprenant en outre l'étape consistant à classer tous les rapports sans confirmation de lecture dans une première liste, les rapports ayant les derniers points temporels en haut de ladite première liste, et tous les autres rapports dans une seconde liste, les rapports ayant les derniers points temporels en haut de ladite seconde liste.

5. Procédé selon l'une quelconque des revendications 1 à 4, le rapport de l'étape e2) comprenant en outre les dates pour des échantillonnages programmés sur la base des dates du point temporel T_{data} de C3).

6. Procédé selon l'une quelconque des revendications 1 à 5, comprenant en outre l'étape consistant à
f) planifier un nouveau cycle de surveillance de la charge pathogène dans le bétail, comprenant les étapes consistant à
f1) recevoir une sélection ou l'entrée de l'exploitation agricole et/ou de la ferme pour laquelle un nouveau cycle doit être planifié,
f2) recevoir une sélection ou l'entrée de la date de début pour le nouveau cycle, et
f3) recevoir une section ou l'entrée de l'âge du bétail au moment du début du nouveau cycle et la portée du nouveau cycle.

7. Procédé selon la revendication 6, l'étape f2) comprenant en outre l'indication des cycles de surveillance de la charge pathogène dans le bétail qui sont actifs.

8. Procédé selon l'une quelconque des revendications 1 à 7, comprenant en outre l'étape consistant à
g) recevoir une demande en provenance du dispositif d'entrée/de sortie d'un utilisateur pour agréger des rapports, comprenant les étapes consistant à
g1) pour toutes les exploitations agricoles et/ou fermes de l'utilisateur, extraire la charge du bétail avec chaque agent pathogène dans chaque exploitation agricole et/ou ferme à partir des données Dₚₐₜ de l'ensemble de données Dᵤₛₑᵣ, former la moyenne de la charge avec chaque agent pathogène, pour obtenir le statut pathogène moyen pour toutes les exploitations agricoles et/ou fermes.

9. Procédé selon l'une quelconque des revendications 1 à 8, comprenant en outre l'étape consistant à
g) recevoir une demande en provenance du dispositif d'entrée/de sortie d'un utilisateur pour agréger des rapports, comprenant les étapes consistant à
g1) pour toutes les exploitations agricoles et/ou fermes de l'utilisateur, extraire la charge du bétail avec chaque agent pathogène dans chaque exploitation agricole et/ou ferme à partir des données Dₚₐₜ de l'ensemble de données Dᵤₛₑᵣ, former la moyenne de la charge avec chaque agent pathogène, pour obtenir le statut pathogène moyen pour toutes les exploitations agricoles et/ou fermes,
g2) extraire la matrice de risque selon la revendication 1 d'une base de données, et
g3) pour toutes les exploitations agricoles et/ou fermes, lire les valeurs v_{L} et/ou v_{T} pour chaque agent pathogène dans les données Dₚₐₜ, et former la moyenne des valeurs v_{L} et/ou v_{T} pour obtenir le risque de condition moyen v_{L,mean} et/ou v_{T,mean} pour toutes les exploitations agricoles et/ou fermes.

10. Produit programme informatique comprenant des parties de code conçues pour exécuter le procédé selon l'une quelconque des revendications 1 à 9, lorsque ledit programme est chargé dans un dispositif informatique.

11. Produit programme informatique stocké sur un support utilisable par ordinateur, comprenant un moyen de programme lisible par ordinateur pour amener un ordinateur à exécuter le procédé selon l'une quelconque des revendications 1 à 9.

12. Système pour surveiller la charge pathogène dans le bétail, comprenant une unité opérationnelle conçue pour exécuter le procédé selon l'une quelconque des revendications 1 à 9, et comprenant ou ayant accès à la base de données avec la matrice de risque selon la revendication 1.
